# EUROPEAN PATENT APPLICATION

(11) **EP 1 336 410 A1**
(43) Date of publication of application: **20.08.2003**
(21) Application number: 01954460.0
(22) Date of filing: 06.08.2001
(51) Int. Cl.: A61K 38/18, A61K 38/32, A61K 39/395, A61K 9/08, A61K 47/10, A61K 47/18, A61K 47/26, A61P 39/00

(54) **PROTEIN INJECTION PREPARATIONS**

(30) Priority: 04.08.2000 JP 2000237432
(71) Applicant: CHUGAI SEIYAKU KABUSHIKI KAISHA, Tokyo, 115-8543 (JP)
(72) Inventor: TANIKAWA, Masahiko, c/o Chugai Seiyaku K. K., Toshima-ku, Tokyo 171-8545 (JP); IIDA, Yoshimitsu, c/o Chugai Seiyaku K. K., Toshima-ku, Tokyo 171-8545 (JP)
(74) Representative: VOSSIUS & PARTNER
(86) International application number: JP0106739
(87) International publication number: WO02011753

(57) **Abstract**

An injectable pharmaceutical formulation containing a physiologically active protein as an active ingredient and at least one sugar as a soothing agent but containing no other proteins as additives and having a pH of 6.5-7.4.

## Description

### TECHNICAL FIELD

The present invention relates to injectable pharmaceutical formulations containing a physiologically active protein as an active ingredient and at least one sugar as a soothing agent but containing no other proteins as additives and having a pH of 6.5-7.4. The present invention also relates to methods for reducing the pain caused by administration of injectable formulations by incorporating at least one sugar into an injectable pharmaceutical composition and adjusting pH to 6.5-7.4.

### BACKGROUND ART

Physiologically active proteins such as erythropoietin, granulocyte colony-stimulating factor (G-CSF) and monoclonal antibodies are unstable and susceptible to extrinsic factors such as temperature, humidity, oxygen, UV rays or the like to undergo physical or chemical changes such as denaturation, aggregation, association, polymerization, oxidation, hydrolysis or disulfide exchange reaction, resulting in great loss of activity.

A conventional means to control these chemical or physical changes is to add proteins commonly used as stabilizers such as human serum albumin. The addition of these proteins such as albumin as stabilizers requires a complicated process to avoid the risk of viral contamination or the like. However, we found that the problem of pain arises from administration of injectable formulations depending on the pH of the solution without adding such proteins as albumin.

On the other hand, physiologically active proteins in solution formulations are relatively more stable under acidic conditions. For example, erythropoietin is known to be stable under acidic conditions around pH 6.0.

However, such acidic conditions may cause pain from administration of injections and therefore should be further controlled.

An object of the present invention is to provide an injectable protein formulation that will cause reduced pain from administration and which has improved stability to allow long-term storage.

Another object of the present invention is to provide a method for reducing the pain caused by administration of injectable formulations by incorporating at least one sugar into an injectable pharmaceutical composition.

### DISCLOSURE OF THE INVENTION

As a result of careful studies, we accomplished the present invention on the basis of the findings that the pain caused by administration is reduced when a sugar is included in place of salts conventionally used as isotonizing agents such as sodium chloride; the pain caused by administration is further reduced at a pH of 6.5-7.4; and the formulations having a pH of 6.5-7.4 and containing the sugar are also sufficiently improved in the stability of a physiologically active protein as the principal agent that they can be stored for an extended period of time.

The present invention provides an injectable formulation containing a physiologically active protein as an active ingredient and at least one sugar as a soothing agent but containing no other proteins as additives and having a pH of 6.5-7.4 (hereinafter referred to as "the formulation of the present invention" or "the present formulation").

The present invention also provides a method for reducing the pain caused by administration of injectable formulations (hereinafter referred to as "the method of the present invention" or "the present method"), comprising incorporating at least one sugar into an injectable pharmaceutical composition (hereinafter referred to as "injectable composition") and adjusting the pH to 6.5-7.4.

Preferred embodiments are as follows.

The present invention provides the injectable formulation as defined above wherein the sugar is at least one member selected from the group consisting of mannitol, sorbitol, trehalose and sucrose.

The present invention provides the injectable formulation as defined above wherein the sugar is mannitol.

The present invention provides the injectable formulation as defined above which has a pH of 6.8-7.2.

The present invention provides the injectable formulation as defined above wherein the physiologically active protein is erythropoietin, granulocyte colony-stimulating factor (G-CSF) or a monoclonal antibody.

The present invention provides the injectable formulation as defined above wherein the physiologically active protein is erythropoietin.

The present invention provides the injectable formulation as defined above which further contains histidine and/or a salt thereof as a stabilizer.

The present invention provides the method for reducing the pain caused by administration of injectable formulations as defined above wherein the sugar is mannitol.

### BRIEF DESCRIPTION OF THE DRAWINGS

FIG. 1 shows analytical results of the difference in pain during injection of drug solutions, comparing the analytical results from formulations B-E (upper panel), pHs 6 and 7 (middle panel) and treatments with mannitol (M) and NaCl (N) (lower panel).
FIG. 2 shows analytical results of the difference in pain after injection of drug solutions, comparing the analytical results from formulations B-E (upper panel), pHs 6 and 7 (middle panel) and treatments with mannitol (M) and NaCl (N) (lower panel).
FIG. 3 shows analytical results of the pain scale during injection of drug solutions, comparing the analytical results from formulations A-E (upper panel), pHs 6 and 7 in contrast with formulation A (middle panel) and treatments with mannitol (M) and NaCl (N) in contrast to formulation A (lower panel).
FIG. 4 shows analytical results of the pain scale after injection of drug solutions, comparing the analytical results from formulations A-E (upper panel), pHs 6 and 7 in contrast to formulation A (middle panel) and treatments with mannitol (M) and NaCl (N) in contrast with formulation A (lower panel).
FIG. 5 shows analytical results of the difference from the pain scale of the first injection during injection of drug solutions, comparing the analytical results from formulations B-E (upper panel), pHs 6 and 7 (middle panel) and treatments with mannitol (M) and NaCl (N) (lower panel).
FIG. 6 shows analytical results of the difference from the pain scale of the first injection after injection of drug solutions, comparing the analytical results from formulations pHs 6 and 7 (upper panel), B-E (middle panel) and treatments with mannitol (M) and NaCl (N) (lower panel).

### THE MOST PREFERRED EMBODIMENTS OF THE INVENTION

As used herein, the "soothing agent" means an additive for reducing the pain caused by administration of injections.

Sugars used as soothing agents in the present invention include not only sugars in a narrow sense consisting of monosaccharides, oligosaccharides and polysaccharides but also sugar alcohols. Sugar alcohols here include not only linear polyhydric alcohols obtained by reduction of carbonyl groups but also cyclic alcohols.

Examples of these sugars include glucose, fructose, saccharose, maltose, lactose, sucrose, mannose, raffinose, mannitol, xylitol, galactitol, glucitol, inositol, sorbitol, trehalose and glycerine. Mannitol, sorbitol, trehalose, sucrose, inositol and glucose are preferably used, more preferably mannitol, sorbitol, trehalose and sucrose, still more preferably mannitol.

The amount of sugars used in the present invention is determined such that the relative osmotic pressure of the present formulation or the relative osmotic pressure of the injectable composition as combined with a sugar in the present method can be each controlled at about 0.5 to about 2.0, preferably about 1. The "relative osmotic pressure" as used here is expressed as a relative ratio based on the physiological osmotic pressure. That is, it means a relative osmotic pressure as determined by calculation for the concentration of sodium chloride, with the relative osmotic pressure of 0.9 w/v % aqueous sodium chloride solution being taken as 1.

Physiologically active proteins used as active ingredients in the present invention include, but not limited to, hematopoietic factors such as granulocyte colony-stimulating factor (G-CSF), granulocyte macrophage colony-stimulating factor (GM-CSF), erythropoietin (EPO) and thrombopoietin; cytokines such as interferon, IL-1 and IL-6; monoclonal antibodies; tissue plasminogen activator (TPA); urokinase; serum albumin; blood coagulation factor VIII; leptin; insulin; and stem cell growth factor (SCF). Preferred proteins are hematopoietic factors such as EPO, G-CSF, GM-CSF and thrombopoietin and monoclonal antibodies, more preferably EPO, G-CSF and monoclonal antibodies.

Physiologically active proteins used as active ingredients in the present invention may be derived from natural sources or preferably genetically engineered so far as they have substantially the same biological activities as those of physiologically active proteins of mammals, especially human. Genetically engineered proteins may have the same amino acid sequences as those of natural proteins or may contain deletion, substitution or addition of one or more amino acids in the amino acid sequences while maintaining the biological activities. Physiologically active proteins also include those chemically modified with PEG or the like.

Physiologically active proteins used as active ingredients in the present invention include, for example, proteins having a sugar chain. The sugar chains may be those derived from any source, but preferably those for glycosylation in mammalian cells. Mammalian cells include, for example, Chinese hamster ovary (CHO) cells, BHK cells, COS cells, human-derived cells, etc., among which CHO cells are most preferred.

When the physiologically active protein used as an active ingredient in the present invention is EPO, EPO may be prepared by any process, e.g. it may be extracted from human urine and isolated and purified by various techniques or may be produced by genetic engineering techniques (see JP-A-61-012288, for example) in Chinese hamster ovary (CHO) cells, BHK cells, COS cells, human-derived cells or the like and then extracted and isolated and purified by various techniques. EPO chemically modified with PEG or the like is also included (see International Publication No. WO90/12874). EPO that has no sugar chain and which has been chemically modified with PEG or the like is also included. EPO analogs are also included, in which EPO has been modified to increase the number of one or more glycosylation sites at the N-linked carbohydrate chain binding site or O-linked carbohydrate binding site in the amino acid sequence of EPO (see JP-A-8-151398 and JP-A-8-506023, for example). Moreover, the amount of sugar chains may be increased by increasing the content of sialic acid or the like without changing the number of sugar chain-binding sites.

When the physiologically active protein used as an active ingredient in the present invention is G-CSF, any highly purified G-CSF can be used. G-CSF in the present invention may be prepared by any process, e.g., they may be extracted from cultures of a human tumor cell line and isolated and purified by various techniques or may be produced by genetic engineering techniques in bacterial cells such as E. coli; yeast cells; animal culture cells such as Chinese hamster ovary (CHO), C127 or COS cells and then extracted and isolated and purified by various techniques. G-CSF is preferably produced by genetic recombination in E. coli, yeast or CHO cells, most preferably by genetic recombination in CHO cells. G-CSF chemically modified with PEG or the like is also included (see International Publication No. WO90/12874).

When the physiologically active protein used as an active ingredient in the present invention is a monoclonal antibody, the monoclonal antibody may be prepared by any process. Monoclonal antibodies can be basically constructed by known techniques as follows. A suitable host is immunized with an immunizing antigen according to a standard immunization technique, and the resulting immunized cells are fused to known parent cells by a standard cell fusion technique, and then the fused cells are screened for monoclonal antibody-producing cells by a standard screening method. Monoclonal antibodies are not limited to those produced by hybridomas, but also include chimeric antibodies obtained by artificial modifications to lower heteroantigenicity to human or for other purposes. Reshaped humanized antibodies can also be used in the present invention, which are obtained by replacing the complementarity-determining regions of a human antibody by the complementarity-determining regions of a non-human mammalian antibody such as a mouse antibody by standard gene recombination techniques also known. These known techniques can be used to obtain reshaped humanized antibodies useful in the present invention.

The protein formulation of the present invention or the injectable composition used in the present method is free from proteins as additives.

As used herein, the "proteins as additives" mean components such as albumin and purified gelatin contained as stabilizers in products currently supplied to the market to control chemical or physical changes of protein formulations.

The present formulation and the injectable composition in the present method may further contain stabilizers. One or more stabilizers may be added in combination.

These stabilizers include amino acids such as histidine, tryptophan, methionine, leucine, phenylalanine, serine, glutamic acid, arginine, lysine and/or salts thereof, among which histidine and/or salts thereof are preferably used.

The amount of stabilizers added to the protein formulation of the present invention and the injectable composition in the present method is typically 0.001-10% (w/v), preferably 0.01-1.0% (w/v).

The formulation of the present invention and the injectable composition in the present method may further contain surfactants. One or more surfactants may be added in combination.

Preferred surfactants are polyoxyethylene sorbitan fatty acid esters, more preferably Polysorbates 20, 21, 40, 60, 65, 80, 81, 85, most preferably Polysorbates 20 and 80.

The amount of surfactants added to the protein formulation of the present invention and the injectable composition in the present method is typically 0.00025-0.5% (w/v), preferably 0.001-0.1% (w/v).

The protein formulation of the present invention and the injectable composition in the present method may further contain diluents, solubilizing agents, pH-modifiers, buffers, sulfur-containing reducing agents, antioxidants, preservatives or the like, if desired. For example, preservatives include quaternary ammonium salts such as benzalkonium chloride and benzethonium chloride; and parabens such as methyl paraoxybenzoate, ethyl paraoxybenzoate, propyl paraoxybenzoate and butyl paraoxybenzoate; which may be used alone or in combination. Sulfur-containing reducing agents include N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and sulfhydryl-containing compounds such as thioalkanoic acid having 1 to 7 carbon atoms. Antioxidants include erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate, sodium metaphosphate. Other components commonly added may also be contained, e.g., inorganic salts such as sodium chloride, potassium chloride, calcium chloride, sodium phosphate, potassium phosphate, sodium bicarbonate; and organic salts such as sodium citrate, potassium citrate, sodium acetate.

Buffers used in the present formulation and the injectable composition in the present method include acids commonly used as buffers in injections and salts thereof or mixed solutions with a base or a salt thereof, such as phosphoric acid, acetic acid, hydrochloric acid, phthalic acid, boric acid, citric acid, carbonic acid, succinic acid and salts thereof, preferably phosphate buffers (sodium monohydrogen phosphate - sodium dihydrogen phosphate system) and/or citrate buffers and/or acetate buffers.

The concentration of buffers used in the protein formulation of the present invention and the injectable composition in the present method is typically 0-300 mM, preferably 0-100 mM on the,basis of the total amount of the present formulation or the injectable composition as combined with a sugar.

The pH of the present formulation and the injectable composition as combined with a sugar in the present method is preferably 6.5-7.4, more preferably 6.8-7.2.

The pH can be adjusted with commonly used pH-modifiers including acids such as hydrochloric acid and bases such as sodium hydroxide.

The protein formulation of the present invention or the physiologically active protein used in the present method is in the form of a solution, a freeze-dried or spray-dried formulation or the like, most preferably a solution formulation.

The protein formulation of the present invention or the injectable composition or physiologically active protein in the present method is normally packed in a sealed and sterilized plastic or glass container, for example. The container can be supplied in the form having a defined volume such as ampules, vials or disposable syringes or a large volume such as injection bags or bottles. Freeze-dried formulations can be dissolved in pure water (water for injection) before use.

Injections of the present invention include drip infusions and are administered subcutaneously, intravenously or intramuscularly, for example.

The concentration of physiologically active proteins used in the present invention can be determined depending on the proteins used, the type of disease to be treated, the severity of the patient, the age of the patient and other factors. Generally, proteins are contained in an amount of 0.5 µg - 100 mg/ml on the basis of the total amount of the present formulation or the injectable composition as combined with a sugar. For example, EPO in a solution formulation is normally contained in an amount of 100-500,000 IU/ml (about 0.5-3000 µg/ml), preferably 200-100,000 IU/ml (about 1-600 µg/ml), more preferably 750-72,000 IU/ml (about 4-400 µg/ml). G-CSF is normally contained in an amount of 1-1000 µg/ml, preferably 10-800 µg/ml, more preferably 50-500 µg/ml expressed as a final administration concentration. When the physiologically active protein is an antibody such as immunoglobulin, monoclonal antibodies and humanized antibodies, it is contained in an amount of 0.1-200 mg/ml, preferably 1-200 mg/ml, more preferably 10-200 mg/ml, most preferably 10-150 mg/ml expressed as a final administration concentration.

The formulation of the present invention is improved in the pain caused by administration and is very stable even after storage at 25°C for 3 months, at 25°C for 6 months and at 40°C for 2 weeks, as shown in the following examples.

The following examples further illustrate the present invention without, however, limiting the scope of the present invention thereto. Various changes and modifications can be made by those skilled in the art on the basis of the description of the present invention, and such changes and modifications are also included in the present invention.

### EXAMPLES

### Example 1: Pain test

The difference in the pain caused by administration to human of samples with varying hydrogen ion concentrations (pHs) and isotonizing agents was examined in comparison with comparative injections which contain sodium chloride as an isotonizing agent or are adjusted to pH 6.

### A. Test method

### Test drugs:

A comparative drug (drug A) and the following samples B-E with varying pHs and isotonizing agents (with the pH and composition of each sample shown in Table 1) were prepared. All the drugs were prepared with the same buffer (phosphate) and the same relative osmotic pressure (1.0), and contain about 10 mmol phosphate, about 2.5 mmol NaCl or about 250 mmol mannitol, 0.1% L-histidine as a stabilizer and 0.005% Polysorbate 80 as an adsorption inhibitor.

**Table 1:**

| List of samples administered | | |
|---|---|---|
| Drug | Formulation details | Relative osmotic pressure |
| A | pH 6.0, phosphate, Tween 80, histidine, NaCl | 1.0 |
| B | pH 6.3, phosphate, Tween 80, histidine, NaCl | 1.0 |
| C | pH 7.0, phosphate, Tween 80, histidine, NaCl | 1.0 |
| D | pH 7.0, phosphate, Tween 80, histidine, mannitol | 1.0 |
| E | pH 6.0, phosphate, Tween 80, histidine, mannitol | 1.0 |

### Administration method of test drugs:

The samples above (Table 1) were paired according to a combination table (Table 2) and administered to the upper arm of each subject (18 normals) at an interval (about 30 seconds) using a syringe containing a predetermined amount (0.5 mL) of each sample (First injection: drug A presumed to cause the strongest pain (a sample of the prior art); Second injection: a sequence of samples according to the combination table). The difference in pain as compared with the first injection and the severity of pain of the first and second injections were recorded.

**Table 2:**

| Combination table for pain test | | |
|---|---|---|
| Subject No. | First injection | Second injection |
| 1 | A | B |
| 2 | A | C |
| 3 | A | B |
| 4 | A | D |
| 5 | A | C |
| 6 | A | D |
| 7 | A | B |
| 8 | A | C |
| 9 | A | B |
| 10 | A | D |
| 11 | A | C |
| 12 | A | D |
| 13 | A | B |
| 14 | A | E |
| 15 | A | C |
| 16 | A | E |
| 17 | A | D |
| 18 | A | E |

### Evaluation items and evaluation criteria:

The following three items were evaluated during and after injection of drug solutions.

### 1. Difference in pain

The severity of the pain caused by the second injection was assessed at 9 scales depending on whether it increased or decreased from the severity of the pain caused by the first injection (drug A) so that in total the severity of pain was assessed at 19 scales; the difference in pain between the first and second injections was recorded under the scale bar at the corresponding scale (Table 3).

**Table 3**

| 1) During injection of drug solutions | | | | | | | | | | | | | | | | | | | |
|---|---|---|---|---|---|---|---|---|---|---|---|---|---|---|---|---|---|---|---|
| | Milder (-) ←← Less Pain than in the first injection | | | | | | | | | First injection | More pain than in the first injection →→Severer (+) | | | | | | | | |
| | 9 | 8 | 7 | 6 | 5 | 4 | 3 | 2 | 1 | 0 | 1 | 2 | 3 | 4 | 5 | 6 | 7 | 8 | 9 |
| Second injection | | | | | | | | | | | | | | | | | | | |

| 2) After injection of drug solutions | | | | | | | | | | | | | | | | | | | |
|---|---|---|---|---|---|---|---|---|---|---|---|---|---|---|---|---|---|---|---|
| | Milder (-) ←← Less Pain than in the first injection | | | | | | | | | First injection | More pain than in the first injection →→Severer (+) | | | | | | | | |
| | 9 | 8 | 7 | 6 | 5 | 4 | 3 | 2 | 1 | 0 | 1 | 2 | 3 | 4 | 5 | 6 | 7 | 8 | 9 |
| Second injection | | | | | | | | | | | | | | | | | | | |

### 2. Pain scale

At each of the first and second administrations, the severity of pain was assessed by the following six criteria and recorded. In the 6-score assessment, the cases of severity shown in Table 4 were rated by numerals 0 - 5 as follows: "no pain", 0; "very mild", 1; "mild", 2; "moderate", 3; "severe", 4; and "unbearable", 5.

The evaluation item of "pain scale" was based on a standard wherein smaller values indicate lower severity of pain. Thus, the smaller the value in each evaluation item, the less the pain caused by the drug solution.

**Table 4**

| 1) During injection of drug solutions | |
|---|---|
| First injection | Second injection |
| No pain | No pain |
| Very mild | Very mild |
| Mild | Mild |
| Moderate | Moderate |
| Severe | Severe |
| Unbearable | Unbearable |

| 2) After injection of drug solutions | |
|---|---|
| First injection | Second injection |
| No pain | No pain |
| very mild | Very mild |
| Mild | Mild |
| Moderate | Moderate |
| Severe | Severe |
| Unbearable | Unbearable |

### 3. Difference from the pain scale of the first injection (drug A)

The difference in evaluation 2 between the first (drug A) and second injections.

### B. Analytical method

[1] Analysis of variance was performed on each evaluation item during and after injection of drug solutions.
[2] Analysis of variance was performed using pH 6.0 for drugs B and E (B was supposed to be at pH 6.0 for analysis) and pH 7.0 for drugs C and D as variant [pH] as well as NaCl for drugs B and C and mannitol for drugs D and E as variant [ISO]. In the analysis of "pain scale", drugs A to E were not totally analyzed but drugs B to E were compared. For drug A, however, the average ± standard error was shown as reference in the graphs without being compared with the other drugs.

### C. Results

### 1. Difference in pain

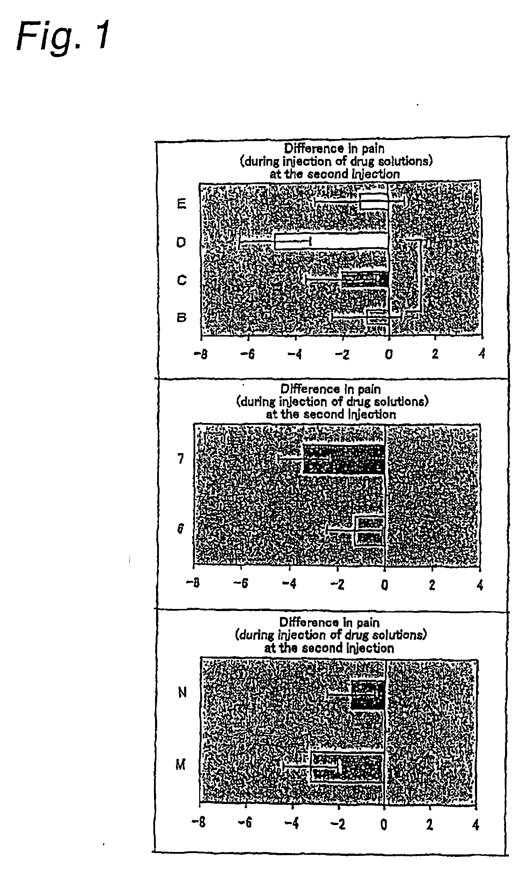

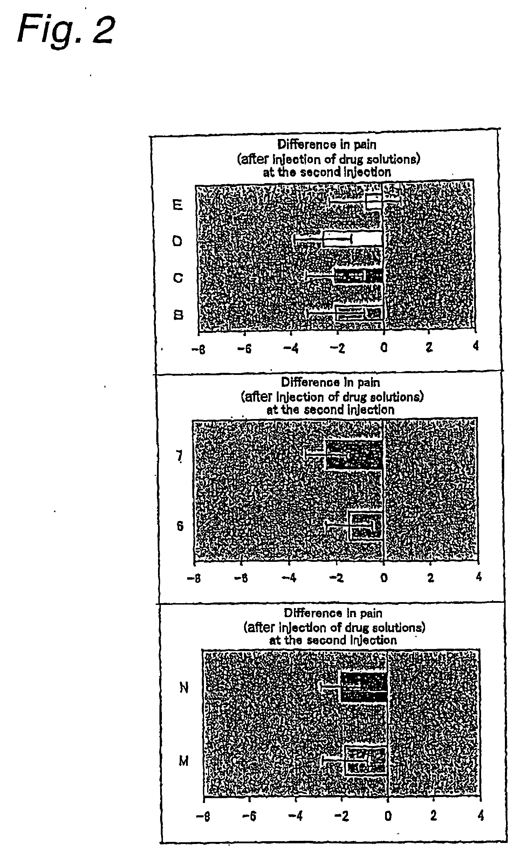

Analytical results of the difference in pain during and after injection of drug solutions are shown in Figs. 1 and 2, respectively.

### 1.1 During injection of drug solutions

[1] The effect of the drug (the main effect of the drug) was studied to show that the severity of the pain caused by drugs B to E was in the order of D<C<B≒E with drug D being improved by about 5 levels on the scale of difference in pain as compared with drug A.
[2] The effect of the pH of the drug solution was studied to show that the pain was less at pH 7 than pH 6 with solutions at pH 7 being improved by about 4 levels on the scale of difference in pain as compared with drug A.
[3] The effect of the isotonizing agent was studied at each stage to show that mannitol caused less pain than NaCl.

### 1.2 After injection of drug solutions

[1] The effect of the drug (the main effect of the drug) was studied to show that the severity of the pain caused by drugs B to E was in the order of D<B≒C≒E with drug D being improved by about 3 levels on the scale of difference in pain as compared with drug A.
[2] The effect of the pH of the drug solution was studied to show that the pain was less at pH 7 than pH 6.
[3] The effect of the isotonizing agent was studied to show that both isotonizing agents brought improvement by about 2 levels on the scale of difference in pain as compared with drug A.

### 2. Pain scale

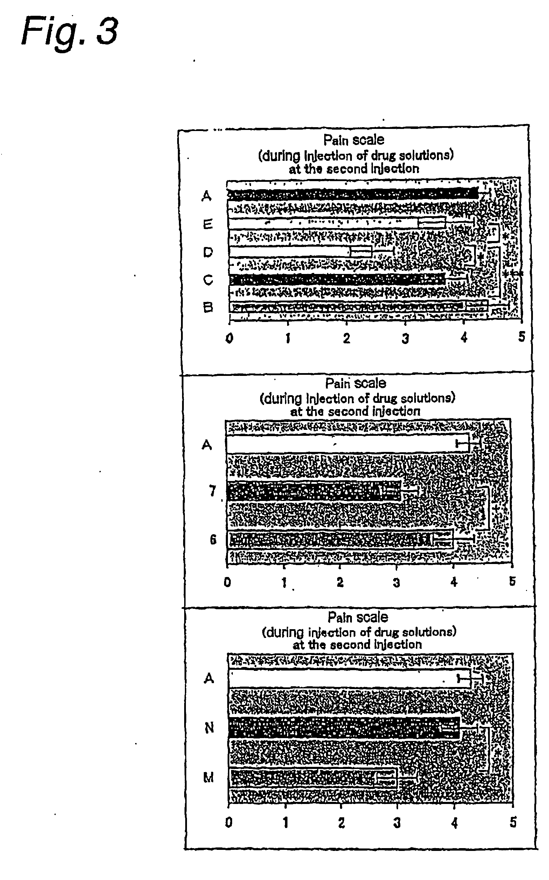

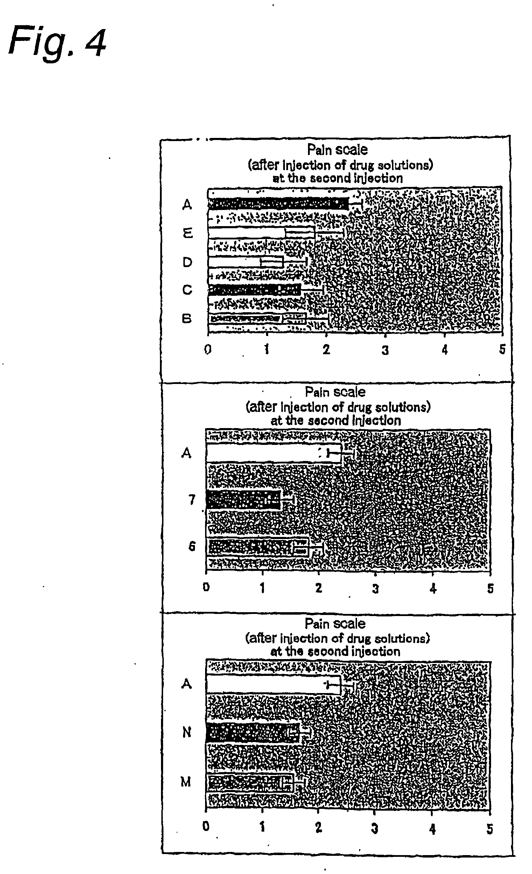

Analytical results of the pain scale during and after injection of drug solutions are shown in Figs. 3 and 4, respectively.

### 2.1 During injection of drug solutions

[1] The effect of the drug was studied to show that the severity of pain was in the order of D<C=E<B with drug D being improved by about 2 levels on the "pain scale" as compared with drug A. Test results showed that drug D was significantly different from any other drugs.
[2] The effect of the pH of the drug solution was studied to show that the pain was less at pH 7 than pH 6 with solutions at pH 7 being improved by about 1 level on the "pain scale" as compared with drug A.
[3] The effect of the isotonizing agent was studied to show that mannitol caused significantly less pain than NaCl.

### 2.2 After injection of drug solutions

[1] The effect of the drug was studied to show that all the drugs were nearly comparable at about 1.5 on the pain scale with D showing a somewhat lower value.
[2] The effect of the pH of the drug solution was studied to show a smaller value at pH 7 than pH 6.
[3] The effect of the isotonizing agent was studied to show that mannitol and NaCl showed nearly comparable values of about 1.5 on the pain scale.

### 3. Difference in pain scale

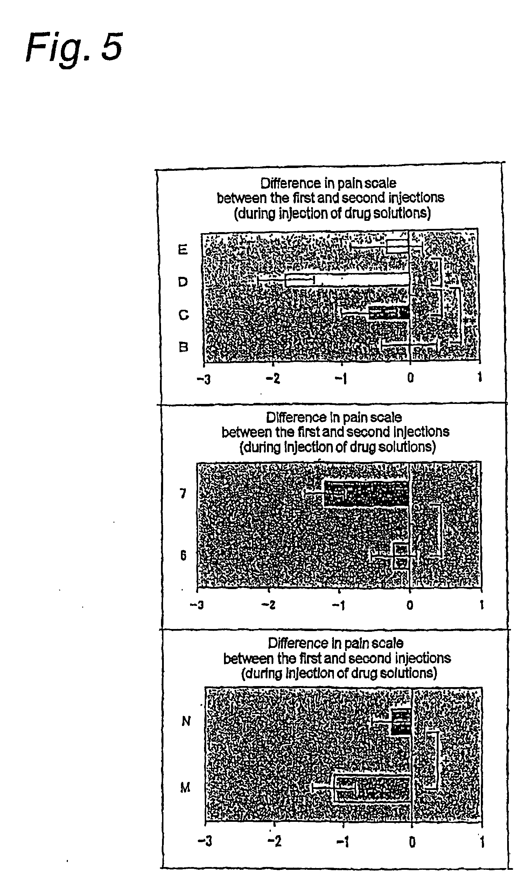

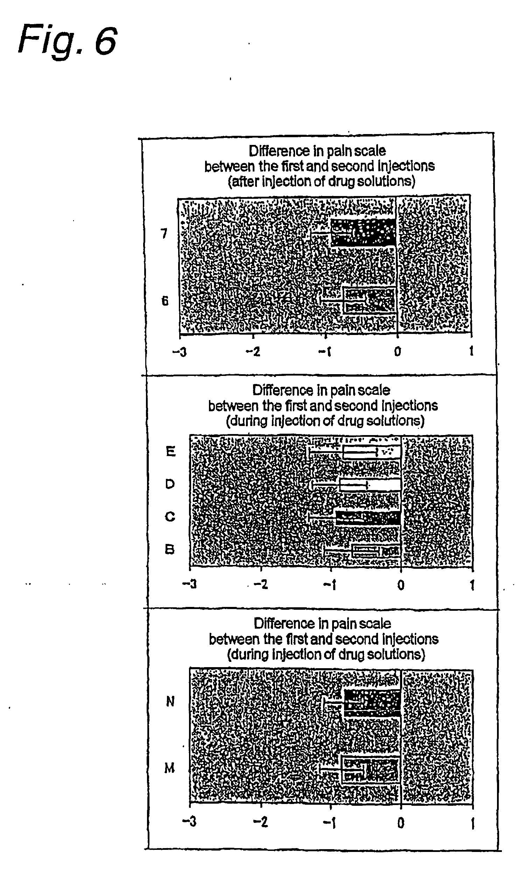

Analytical results of the difference in pain scale between the first (drug A) and second injections during and after injection of drug solutions are shown in Figs. 5 and 6, respectively.

### 3.1 During injection of drug solutions

[1] The effect of the drug was studied to show that the severity of pain was in the order of D<C<E<B.
[2] The effect of the pH of the drug solution was studied to show that the pain was significantly less at pH 7 than pH 6, similarly to the "difference in pain".
[3] The effect of the isotonizing agent was studied to show that mannitol caused significantly less pain than NaCl.

### 3.2 After injection of drug solutions

[1] The effect of the drug was studied to show a difference in pain scale of -1 or less in each of the drugs.
[2] The effect of the pH of the drug solution was studied to show that the results were nearly comparable at both pHs with the difference in "pain scale" being less than -1.
[3] The effect of the isotonizing agent was studied to show that the effects of mannitol and NaCl were nearly comparable with the difference in "pain scale" being less than -1.

### E. Discussion

1) As to the influence of the hydrogen ion concentration (pH) on the pain during injection of drug solutions, the severity of pain was clearly lower at pH 7.0 than pH 6.0.
2) As to the influence of the isotonizing agent on the pain during injection of drug solutions, mannitol was excellent.
3) Formulation D was the most excellent in the pain after injection of drug solutions within the range of this test, but all the formulations including formulation A caused mild pain with no practical problem.

### Example 2: Stability test

Erythropoietin solution formulations with varying formulation factors found to influence pain in Example 1 (pH and/or isotonizing agent) were prepared as test samples and evaluated for stability by quantification and purity assays.

Specifically, stability was compared between formulations containing D-mannitol (hereinafter referred to as "Man added") and formulations containing sodium chloride (hereinafter referred to as "Man not added") because the result of the pain test on normals in Example 1 showed that the pain caused by injection of drug solutions was relieved when D-mannitol was used as an isotonizing agent as compared with sodium chloride.

Stability of erythropoietin solutions under weak acid to weak alkaline conditions was also examined because the pain was relieved at pH 7.0 as compared with pH 6.0.

### A. Test samples

All the samples used in formulation studies contain 750 IU erythropoietin in 0.5 mL. The samples tested are shown in Table 5. All the samples used were prepared as erythropoietin (genetically engineered EPO) solution formulations (1500 IU/ml) containing L-histidine (0.1%) as a stabilizer and Polysorbate 80 (0.005%) using the same buffer (phosphate) and the same relative osmotic pressure (1.0). They contain about 10 mmol phosphate and about 2.5 mmol NaCl or about 250 mmol D-mannitol.

**Table 5:**

| Components of test samples | |
|---|---|
| Formulation | pH, buffer, adsorption inhibitor, stabilizer, isotonizing agent |
| A | pH6.0, phosphate, Tween 80, histidine, NaCl |
| B | pH6.5, phosphate, Tween 80, histidine, NaCl |
| C | pH7.0, phosphate, Tween 80, histidine, NaCl |
| D | pH7.5, phosphate, Tween 80, histidine, NaCl |
| E | pH6.0, phosphate, Tween 80, histidine, mannitol |
| F | pH7.0, phosphate, Tween 80, histidine, mannitol |

### B. Test method

Each formulation test sample was stored at 25°C for 6 months and evaluated by purity assay 1 [product-related impurities: aggregates (SDS-PAGE)], purity assay 2 [product-related impurities: low molecular weight degradation products (SDS-PAGE(R)] and quantification [liquid chromatography (HPLC)].

### 1. Purity assay 1 [product-related impurities: aggregates (SDS-PAGE)]

### (1) Test method

Each sample solution, a standard solution and a molecular weight marker solution were electrophoresed and detected by Western blotting.

### 2. Purity assay 2 [product-related impurities: low molecular weight degradation products (SDS-PAGE(R)]

### (1) Test method

A 7.5 µL aliquot of each sample was electrophoresed and detected by Western blotting using an anti-erythropoietin antibody (prepared as a polyclonal antibody).

The band detected around 32 kd was read with a densitometer to calculate the concentration from the staining strength of the sample.

### 3. Quantification [liquid chromatography (HPLC)]

### (1) Test method

Liquid chromatography was used to determine the content of erythropoietin (% of the nominal potency) from the peak area of erythropoietin.

### 4. Discussion and/or Conclusion

### [Influence of pH on the stability of erythropoietin solutions]

Table 6 shows the relation between pH and the residual rate by HPLC of erythropoietin solutions (1500 IU/mL) after storage at 25°C for 6 months (the results of formulations A-D). Taking into account of variation in measurement results, the residual rate by HPLC remained unchanged at pH 6.0-7.0 and decreased at pH 7.5.

**Table 6:**

| Influence of pH on the stability of erythropoletin solutions (1500 IU/mL) | | | | |
|---|---|---|---|---|
| pH | 6.0 | 6.5 | 7.0 | 7.5 |
| Residual rate (%) by HPLC after storage at 25°C for 6 months | 94.9 | 93.3 | 92.4 | 89.3 |

### [Influence of sugar on the stability of erythropoietin solutions]

Table 7 shows the relation between Man addition and the residual rate of erythropoietin solutions (1500 IU/mL) by HPLC after storage at 25°C for 6 months (the results of formulations E, F compared with formulations A, C). Although any formulations at both pH 6.0 and pH 7.0 had no problem with stability, the formulation containing no Man at pH 6.0 and the formulation containing Man at pH 7.0 showed somewhat higher contents by HPLC.

**Table 7:**

| Influence of sugar on the stability of erythropoietin solutions (1500 IU/mL) | | |
|---|---|---|
| Residual rate (%) by HPLC after storage at 25°C for 6 months | pH6.0 | pH7.0 |
| Man not added | 94.9 | 92.4 |
| Man added | 93.3 | 94.0 |

### [Conclusion]

The foregoing results show that formulation F, which is an erythropoietin solution formulation having a pH of 7.0 and containing D-mannitol as an isotonizing agent, is the most preferred in the pain reduction caused by injection and the stability of the formulation.

Comparison of the stability of formulations F and A after storage at 25°C for 6 months revealed that formulation F was comparable to formulation A in both residual rate and degradation species.

It was concluded from this result that formulation F should be stably stored at 10°C for 2 years or more as in the case of formulation A which is known to be stable at 10°C for 2 years or more.

## Claims

1. An injectable pharmaceutical formulation containing a physiologically active protein as an active ingredient and at least one sugar as a soothing agent but containing no other proteins as additives and having a pH of 6.5-7.4.

2. The injectable formulation of Claim 1 wherein the sugar is at least one member selected from the group consisting of mannitol, sorbitol, trehalose and sucrose.

3. The injectable formulation of Claim 1 wherein the sugar is mannitol.

4. The injectable formulation of any one of Claims 1 to 3 which has a pH of 6.8-7.2.

5. The injectable formulation of any one of Claims 1 to 4 wherein the physiologically active protein is erythropoietin, granulocyte colony-stimulating factor or a monoclonal antibody.

6. The injectable formulation of Claim 5 wherein the physiologically active protein is erythropoietin.

7. The injectable formulation of Claim 6 which is a yet to be freeze-dried erythropoietin solution formulation.

8. The injectable formulation of any one of Claims 1 to 7 which further contains histidine and/or a salt thereof as a stabilizer.

9. A method for reducing the pain caused by administration of injectable formulations, comprising incorporating at least one sugar into an injectable pharmaceutical composition and adjusting the pH to 6.5-7.4.

10. The method of Claim 9 wherein the sugar is mannitol.
